# EUROPEAN PATENT APPLICATION

(11) **EP 2 453 255 A1**
(43) Date of publication of application: **16.05.2012**
(21) Application number: 11187787.4
(22) Date of filing: 04.11.2011
(51) Int. Cl.: G01S 7/52, G01S 15/89, A61B 8/14

(54) **Ultrasound system and method for providing preview image**

(30) Priority: 11.11.2010 KR 20100111822
(71) Applicant: Samsung Medison Co., Ltd., Nam-myun Hongchun-gun Kangwon do 250-875 (KR)
(72) Inventor: Kim, Yun Jin, 135-851 Seoul (KR)
(74) Representative: Schmid, Wolfgang

(57) **Abstract**

There is disclosed an embodiment for providing a preview image. An ultrasound data acquisition unit (110) transmits and receives ultrasound signals to and from a target object to acquire a plurality of ultrasound data. A processor (130) forms volume data (510) by using the plurality of ultrasound data. The processor (130) further sets a plurality of rendering directions (610,621-623) corresponding to a plurality of geometries and renders the volume data (510) along the plurality of respective rendering directions (610,621-623) to form a plurality of preview images.

## Description

### CROSS-REFERENCE TO RELATED APPLICATIONS

The present application claims priority from Korean Patent Application No. 10-2010-0111822 filed on November 11, 2010.

### TECHNICAL FIELD

The present invention generally relates to ultrasound systems, and more particularly to an ultrasound system and method for providing a preview image.

### BACKGROUND

An ultrasound system has become an important and popular diagnostic tool due to its non-invasive and non-destructive nature. The ultrasound system can provide high dimensional real-time ultrasound images of inner parts of target objects without any surgical operation.

The ultrasound system may provide a three-dimensional ultrasound image including clinical information such as spatial information and anatomical figures of the target objects, which cannot be provided by a two-dimensional ultrasound image. Generally, the ultrasound system may transmit ultrasound signals to a target object, receive ultrasound echo signals reflected from the target object and form volume data by using the received ultrasound echo signals. The ultrasound system may render the volume data along a predetermined rendering direction to thereby form the three-dimensional ultrasound image.

Conventionally, the three-dimensional ultrasound image is formed by rendering the volume data along the predetermined rendering direction. As a result, there is a disadvantage since it is required to rotate or move the three-dimensional ultrasound image in a plurality of directions to search for the three-dimensional ultrasound image corresponding to a desirable view.

### SUMMARY

An embodiment for providing a preview image is disclosed herein. In one embodiment, by way of non-limiting example, an ultrasound system may include: an ultrasound data acquisition unit configured to transmit and receive ultrasound signals to and from a target object to acquire a plurality of ultrasound data; and a processor coupled to the ultrasound data acquisition unit and being configured to form volume data by using the plurality of ultrasound data, set a plurality of rendering directions corresponding to a plurality of geometries and render the volume data along the plurality of respective rendering directions to form a plurality of preview images.

In another embodiment, a method of providing a preview image may comprise: a) transmitting and receiving ultrasound signals to and from a target object to output a plurality of ultrasound data; b) forming volume data by using the plurality of ultrasound data; c) setting a plurality of rendering directions corresponding to a plurality of geometries; and d) rendering the volume data along the plurality of respective rendering directions to form a plurality of preview images.

This Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a schematic diagram showing an example of a scanning direction to obtain frames.
FIG. 4 is a flow chart showing an illustrative embodiment of method for providing a plurality of preview images.
FIG. 5 is a schematic diagram showing an example of volume data.
FIG. 6 is a schematic diagram showing an example of rendering directions.

### DETAILED DESCRIPTION

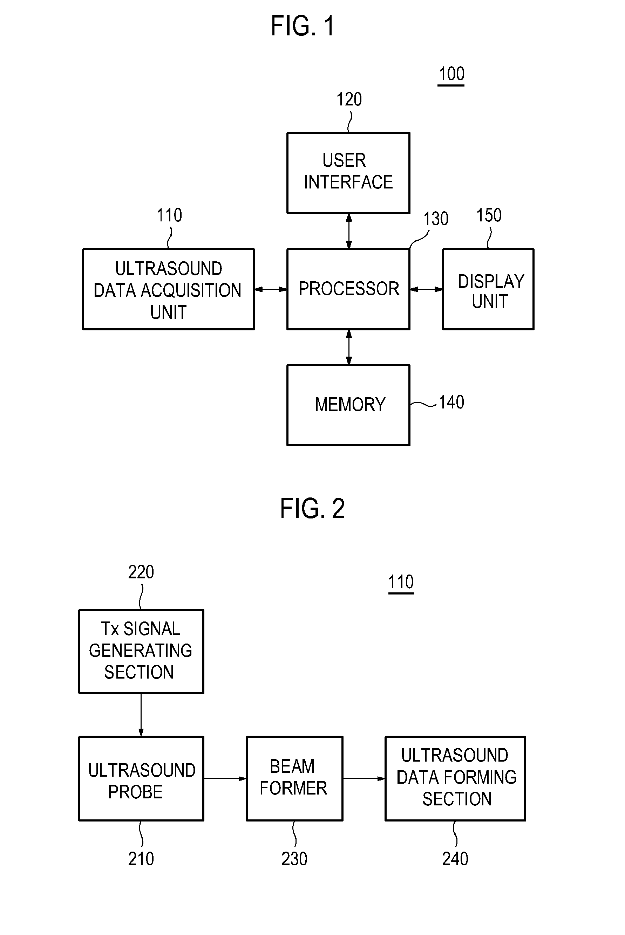

This detailed description is provided with reference to the accompanying drawings. One of ordinary skill in the art may realize that the following description is illustrative only and is not in any way limiting. Other embodiments of the present invention may readily suggest themselves to such skilled persons having the benefit of this disclosure. FIG. 1 is a block diagram showing an illustrative embodiment of an ultrasound system. As depicted therein, the ultrasound system 100 includes an ultrasound data acquisition unit 110, a user interface 120, a processor 130, a memory 140 and a display unit 150. The ultrasound data acquisition unit 110 is configured to transmit and receive ultrasound signals to and from a target object to thereby form ultrasound data. FIG. 2 is a block diagram showing an illustrative embodiment of the ultrasound data acquisition unit 110. Referring to FIG. 2, the ultrasound data acquisition unit 110 includes an ultrasound probe 210, a transmit (Tx) signal generating section 220, a beam former 230 and a ultrasound data forming section 240.

The ultrasound probe 210 includes a plurality of transducer elements (not shown) for reciprocally converting between electrical signals and ultrasound signals. The ultrasound probe 210 transmits ultrasound signals to the target object and receives ultrasound echo signals reflected from the target object to thereby form the received signals. The received signals are analog signals. The ultrasound probe 210 includes a three dimensional mechanical probe, a 2D array probe and the like.

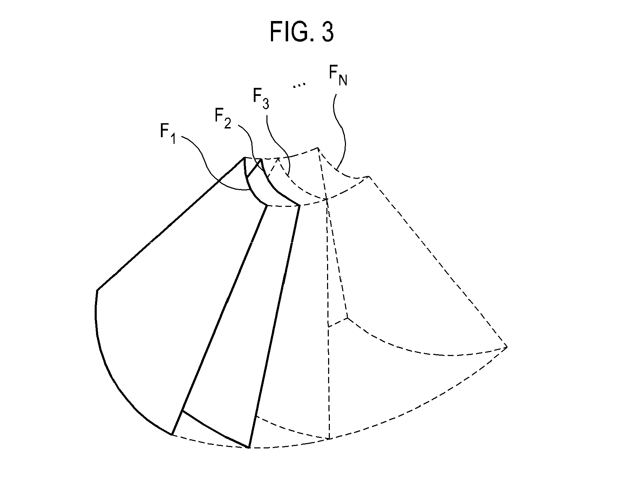

The Tx signal generating section 220 is configured to control transmission of the ultrasound signals. Furthermore, the Tx signal generating section 220 generates Tx signals to acquire frames in consideration of distances between the respective transducer elements and focal points. In one embodiment, the Tx signal generating section 220 generates Tx signals to acquire a plurality of respective frames Fi (1≤i≤N) as depicted in FIG. 3. Accordingly, when the Tx signals are provided from the Tx signal generating section 220, the ultrasound probe 210 converts the Tx signals into the ultrasound signals, transmit the ultrasound signals to the target object and receive ultrasound the echo signals reflected from the target object to thereby form the received signals.

The beam former 230 converts the received signals provided from the ultrasound probe 210 into digital signals. Furthermore, the beam former 126 applies delays to the digital signals in consideration of distances between the transducer elements and focal points to thereby output receive-focused signals. In one embodiment, the beam former 126 converts a plurality of received signals sequentially provided form the ultrasound probe 210 into a plurality of digital signals. Furthermore, the beam former 126 applies delays to the plurality of respective digital signals in consideration of distances between the transducer elements and focal points to thereby form a plurality of receive-focused signals.

The ultrasound data forming section 240 forms the ultrasound data by using the receive-focused signals provided from the beam former 230. The ultrasound data includes radio frequency (RF) data. However, it should be noted herein that the ultrasound data may not be limited thereto. Furthermore, the ultrasound data forming section 240 performs a variety of signal processing, i.e. gain control, on the receive-focused signals. In one embodiment, the ultrasound data forming section 240 forms the ultrasound data corresponding to the plurality of respective frames Fᵢ (1≤i≤N) by using the receive-focused signals sequentially provided from the beam former 230. Referring back to FIG. 1, the user interface 120 receives input information from a user. In one embodiment, the input information includes select information for selecting at least one preview image among a plurality of preview images. However, it should be noted herein that the input information may not be limited thereto. The user interface 120 includes a control panel, a trackball, a mouse, a keyboard and the like.

The processor 130 is connected to the ultrasound data acquisition unit 110 and the user interface 120. The processor 130 renders the volume data along a plurality of different rendering directions to form a plurality of preview images corresponding to a plurality of views.

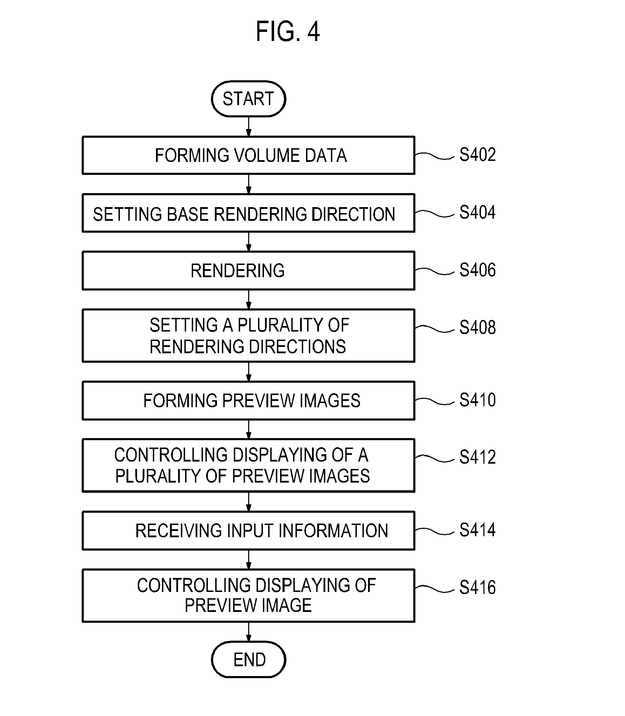

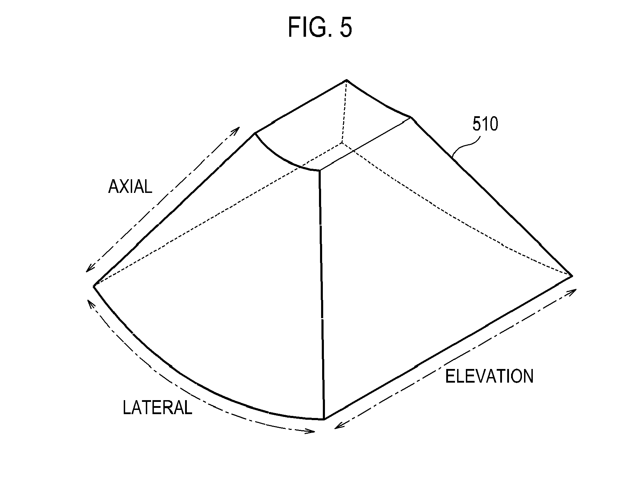

FIG. 4 is a flow chart showing an illustrative embodiment of method for providing the plurality of preview images. Referring to FIG. 4, the processor 130 forms the volume data 510 as shown in FIG. 5 by using a plurality of ultrasound data provided from the ultrasound data acquisition unit 110, at step S402. The volume data 510 are stored in the memory 140.

FIG. 5 is a schematic diagram showing an example of the volume data. The volume data 510 includes a plurality of voxels (not shown) each having a brightness value. Referring to FIG. 5, an axial direction represents a propagation direction of ultrasound signals from the transducer elements of the ultrasound probe 210, a lateral direction represents a moving direction of a scanline and an elevation direction represents a scanning direction for the frames (i.e., scanning planes), which is a depth direction of the three-dimensional ultrasound image.

Referring back to FIG. 4, the processor 130 sets a reference rendering direction for forming the three-dimensional ultrasound image, at step S404. The processor 130 renders the volume data 510 along the set reference rendering direction to form the three-dimensional ultrasound image, at step S406.

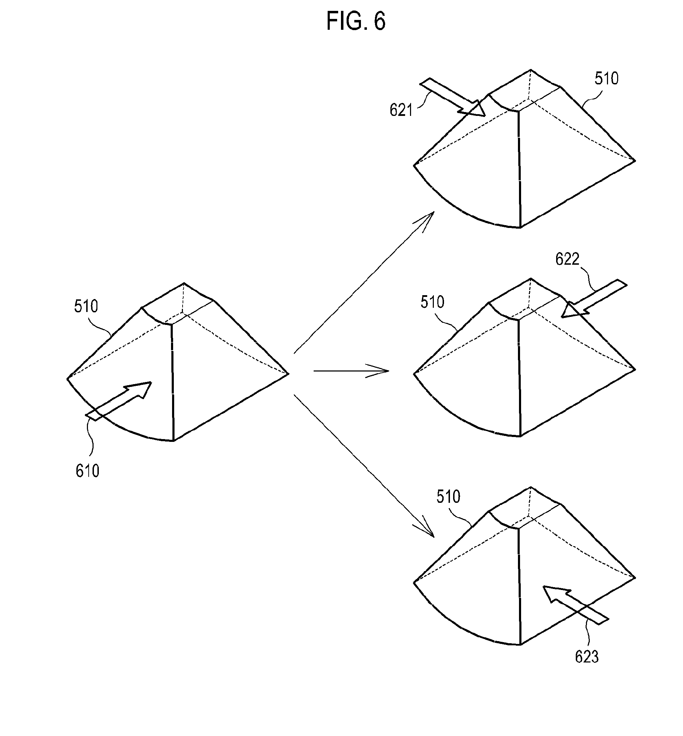

The processor 130 sets a plurality of rendering directions corresponding to a plurality of geometries based on the reference rendering direction, at step S408. In one embodiment referring to FIG. 6, the processor 130 sets the first rendering direction 621 by rotating 90 degrees from the reference rendering direction 610, the second rendering direction 622 by rotating 180 degrees from the reference rendering direction 610 and the third rendering direction 623 by rotating 270 degrees from the reference rendering direction 610.

The number of rendering directions may not be limited thereto. The processor 130 sets a plurality of rendering directions corresponding to x, y and z axes of three-dimensional Cartesian coordinates, respectively, based on the reference rendering direction.

The processor 130 renders the volume data along the plurality of rendering directions to form the plurality of preview images corresponding to the plurality of rendering directions, at step S410. The preview images include the three-dimensional ultrasound images.

The processor 130 controls display of the plurality of preview images, at step S412. In one embodiment, the processor 130 divides a screen region of the display unit 150 into a plurality of division regions and displays the plurality of preview images on the division regions. Therefore, a user selects at least one preview image among the plurality of preview images by using the user interface 120.

When the input information is provided from the user interface 120, at step S414, the processor 130 controls display of the preview image corresponding to the input information among the plurality of preview images, at step S416. In one embodiment, the processor 130 controls display of the preview image corresponding to the input information only. In another embodiment, the processor 130 controls displaying of enlarged preview image corresponding to the input information only.

Alternatively, the processor 130 sets the rendering direction of the preview image corresponding to the input information as the reference rendering direction.

Referring back to FIG. 1, the memory 140 stores the plurality of ultrasound data acquired by the ultrasound data acquisition unit 110. Furthermore, the memory 140 stores the volume data formed by the processor 130.

The display unit 150 displays the plurality of preview images formed by the processor 130. Furthermore, the display unit 150 displays the preview image corresponding to the input information provided from the user interface 120. Furthermore, the display unit 150 displays a reference three-dimensional ultrasound image formed by the processor 130.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other embodiments.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit and receive ultrasound signals to and from a target object to acquire a plurality of ultrasound data; and
a processor coupled to the ultrasound data acquisition unit and being configured to form volume data by using the plurality of ultrasound data, set a plurality of rendering directions corresponding to a plurality of geometries and render the volume data along the plurality of respective rendering directions to form a plurality of preview images.

2. The ultrasound system of Claim 1, wherein the processor being configured to:
set a reference rendering direction of the volume data; and
set the plurality of rendering directions based on the reference rendering direction.

3. The ultrasound system of Claim 1, further comprising:
a user interface configured to receive input information for selecting at least one preview image among the plurality of preview images.

4. A method of providing a preview image, comprising:
a) transmitting and receiving ultrasound signals to and from a target object to output a plurality of ultrasound data;
b) forming volume data by using the plurality of ultrasound data;
c) setting a plurality of rendering directions corresponding to a plurality of geometries; and
d) rendering the volume data along the plurality of respective rendering directions to form a plurality of preview images.

5. The method of Claim 4, wherein the step c) comprises:
setting a reference rendering direction of the volume data; and
setting the plurality of rendering directions based on the reference rendering direction.

6. The method of Claim 4, further comprising:
receiving input information for selecting at least one preview image among the plurality of preview images.
